# EUROPEAN PATENT APPLICATION

(11) **EP 2 896 421 A1**
(43) Date of publication of application: **22.07.2015**
(21) Application number: 14199187.7
(22) Date of filing: 18.04.2008
(51) Int. Cl.: A61M 5/31

(54) **Ergonomic syringe**

(30) Priority: 20.04.2007 US 913198 P
(62) Divisional of application: 08746321.2
(71) Applicant: Barbour, Jennifer, Denver, CO 80222 (US)
(72) Inventor: Barbour, Jennifer, Denver, CO 80222 (US)
(74) Representative: Hanson, William Bennett

(57) **Abstract**

A syringe comprises a barrel portion (20) and a plunger portion (30) comprising a distal end adapted for insertion into a proximal end of the barrel portion (20). A gripping device (160) is positioned on a proximal end (31) of the plunger portion. The gripping device comprises an open loop portion that is sized and configured to operatively receive a user's thumb and to facilitate one-handed aspiration during operation of the syringe.

## Description

### Reference to Related Application

The present application claims priority to U.S. provisional application No. 60/913,198, filed April 20, 2007, the entirety of which is hereby incorporated by reference.

### Field of the Invention

A device and method relating to injection devices and methods are disclosed in accordance with preferred embodiments of the present invention. In some embodiments, the device and method provide for ease of use of injection devices.

### Background

Devices for delivery or injection of substances, such as syringes, are well-known. Despite the importance of such devices and the fact that such devices are being used with ever increasing frequency, the art has seen little in the way of developments in regard to certain aspects of these injection devices.

### SUMMARY OF THE INVENTION

While much emphasis is placed on the comfort of a subject that is to receive an injection via a syringe, frequently, the comfort of the person using the syringe is ignored. In some of the embodiments described herein, it has been appreciated that the user's comfort can be important. This can be especially relevant when numerous injections, small volumes to be injected, complex procedures (such as aspiration), or any combination thereof occur. Thus, in some embodiments, an ergonomic syringe can be employed for ease of use. In some embodiments, this is especially advantageous when the substance to be injected is a dermal filler.

In addition, in some embodiments, there is a significant need for devices and systems for one-handed aspiration in conjunction with injection. Preferably, such devices and systems for one handed aspiration and injection can be configured to reduce the risk of injury. It is therefore desirable to provide a convenient, accurate, comfortable system for one handed aspiration and injection.

In some embodiments, the present invention concerns devices and systems for aspiration and injection, especially a device for one-handed aspiration and injection.

A syringe is disclosed in accordance with some embodiments of the present invention. In some embodiments, the syringe has a barrel portion, a cushion positioned against said barrel portion, wherein the cushion is configured to provide cushioning when said barrel portion is operably grasped by two adjacent fingers of a same hand, and a plunger portion having a distal end adapted for insertion into a proximal end of said barrel portion.

In some embodiments, the syringe has a predefined volume of a dermal filler contained within the barrel portion. In some embodiments, the syringe is configured to inject a total volume of less than about 2 cc. In some embodiments, the syringe is configured to inject a total volume of less than about 1 cc. In some embodiments, the cushion is integral to the syringe. In some embodiments, the cushion is detachable from the syringe.

In some embodiments, the syringe has a second cushion positioned adjacent to a proximal end of the plunger portion. In some embodiments, the syringe has a thumb grip positioned on a proximal end of the plunger portion. In some embodiments, an outer diameter of the barrel portion of the syringe is less than about 1 cm.

In some embodiments the thumb grip has a broken loop positioned to facilitate one-handed aspiration during operation. In some embodiments the syringe has a third cushion positioned on the thumb grip and configured to provide cushioning during one-handed aspiration. In some embodiments, the thumb grip is integral to the syringe. In other embodiments, the thumb grip is detachable from the syringe.

In some embodiments, a cushion is positioned against the barrel portion of the syringe and completely encircles the barrel portion. In some embodiments, a cushion positioned against the barrel portion has a substantially circular cross-sectional area encircling the barrel portion. In some embodiments, a cushion positioned against the barrel portion has a generally oblong cross-sectional area encircling the barrel portion and has an upper lip, a lower lip, or an upper lip and a lower lip. In some embodiments at least a portion of the cushion positioned against the barrel portion extends at least 0.5 cm beyond the circumference of the barrel portion.

In some embodiments, the syringe is configured to inject multiple, pre-measured amounts of an injectable substance, wherein the pre-measured amount is about 0.1 cc. In some embodiments, the syringe has stops to indicate when a pre-measured amount of fluid has been injected, and the stops (e.g., indicators) are visible to an operator when the syringe is gripped between two adjacent fingers.

In some embodiments, the syringe has a flange located on a proximal portion of the barrel portion. In some embodiments, the syringe has a fourth cushion located adjacent to a flange on a proximal portion of the barrel portion, and the cushion is configured to provide cushioning when force is exerted against the flange.

In some embodiments, any one of the cushions of the syringe is integral to the syringe. In some embodiments, any one of the cushions of the syringe is detachable from the syringe.

In some embodiments, the syringe is sterilizable.

A kit is disclosed in accordance with some embodiments of the present invention. The kit has at least two syringes, wherein each syringe has a barrel portion, a cushion positioned against the barrel portion, wherein the cushion is configured to provide cushioning when the barrel portion is operably grasped by two adjacent fingers of a same hand, a plunger portion having a distal end adapted for insertion into a proximal end of the barrel portion, and a specified amount of a dermal filler contained within the barrel portion. In some embodiments, the barrel portion contains about 1 cc or less of dermal filler.

A kit for injecting a dermal filler is disclosed in accordance with some embodiments of the present invention. The kit has at least one syringe. Each syringe has a barrel portion and a plunger portion having a distal end adapted for insertion into a proximal end of the barrel portion, a cushion configured to be positioned against the barrel portion and to provide cushioning when the barrel portion is operably grasped by two adjacent fingers of a same hand, and a specified amount of a dermal filler contained within said barrel portion. In some embodiments, a method of manipulating a syringe is provided. The method can include gripping a barrel portion of a syringe with two adjacent fingers of a same hand, wherein at least one cushion is positioned between at least one of the fingers and the barrel portion of the syringe and injecting a dermal filler.

In some embodiments, a syringe is provided. The syringe comprises, consists, or consists essentially of a barrel portion, a plunger portion comprising a distal end adapted for insertion into a proximal end of the barrel portion, and a thumb grip. The thumb grip can be positioned on a proximal end of said plunger portion. The thumb grip can comprise a strap. The strap can comprise a flexible, elastic, or flexible and elastic material. In some embodiments, the thumb grip is detachable from the syringe. In some embodiments, the strap comprises an elastic material. In some embodiments, the strap comprises, consists, or consists essentially of a first hole at a first end of the strap and a second hole at a second end of the strap. In some embodiments, at least a portion of the plunger portion is passed through both the first and second holes of the strap. In some embodiments, the proximal end of the plunger portion is larger in diameter than the diameter of the first, second, or first and second hole(s). In some embodiments, the strap comprises a material selected from the group consisting of rubber, nylon, cotton, foam rubber, neoprene, plastic, or any combination thereof. In some embodiments, the syringe further comprises a cushion positioned against said barrel portion. The cushion can be configured to provide cushioning when said barrel portion is operably grasped by two adjacent fingers of a same hand. In some embodiments, said thumb grip is integral to the syringe. In some embodiments, the strap is sized so as to allow a user's finger or thumb to fit between the proximal end of the plunger portion and a surface of the strap without stretching the strap. In some embodiments, the strap is sized so as to require the strap to be stretched in order to allow a user's finger or thumb to fit between the proximal end of the plunger portion and a surface of the strap. In some embodiments, the thumb grip consists essentially of a flexible, elastic, or flexible and elastic strap. In some embodiments, the strap consists essentially of a first hole at a first end of the strap and a second hole at a second end of the strap.

In some embodiments, a kit is provided. The kit comprises, consists, or consists essentially of a syringe comprising a barrel portion and a plunger portion comprising a distal end adapted for insertion into a proximal end of the barrel portion. The plunger portion further comprises a proximal end. The proximal end is larger in diameter than the distal end of the plunger portion. The kit further comprises, consists, or consists essentially of a strap comprising consisting, or consisting essentially of a first hole at a first end and a second hole at a second end of the strap. The strap comprises, consists, or consists essentially of a flexible, elastic, or flexible and elastic material. In some embodiments, the kit further comprises a cushion positioned against said barrel portion, wherein the cushion is configured to provide cushioning when said barrel portion is operably grasped by two adjacent fingers of a same hand. In some embodiments, strap is elastic.

In some embodiments, a method of making a syringe is provided. The method comprises, consists, or consists essentially of providing a barrel portion, a plunger portion, and a strap comprising, consisting, or consisting essentially of a first hole at a first end of the strap and a second hole at a second end of the strap. The strap comprises a flexible, elastic, or flexible and elastic material. One can insert a distal end of the plunger portion through said first hole of the strap. One can insert the distal end of the plunger portion through said second hole of the strap. One can insert said distal end of the plunger portion into a proximal end of the barrel portion.

In some embodiments, a method of using a syringe is provided. The method comprises, consists, or consists essentially of providing a syringe comprising a barrel portion and a plunger portion comprising a distal end adapted for insertion into a proximal end of the barrel portion, providing a strap that is looped around a proximal end of the plunger portion. The strap comprises, consists, or consists essentially of a first hole and a second hole. At least a part of the plunger portion is passed through the first hole and second hole. The strap is sized so as to allow a finger or thumb to be inserted between the proximal end of the plunger portion and the strap. The strap comprises, consists, or consists essentially of a flexible, elastic, or flexible and elastic material. One can insert a finger or thumb between the proximal end of the plunger and the strap, and one can aspirate the syringe by exerting a force against the strap via the finger or thumb. In some embodiments, the insertion of the finger or thumb between the proximal end of the plunger and the strap stretches the strap. In some embodiments, the stretched strap exerts a constricting force on the finger or thumb. In some embodiments, the strap, when not stretched, is between 2 and 10 cm in length. In some embodiments, the strap, when stretched, is between 2 and 10 cm in length.

These and other embodiments are described in greater detail below.

### BRIEF DESCRIPTION OF THE DRAWINGS

**FIG. 1** depicts a syringe 10 having a cushion 50 positioned against the barrel portion 20, a plunger portion 30, a cushioned thumb cushion 60, and a flange 40.
**FIG. 2** depicts a barrel portion 20 having a cushion 90 and a flange 40 on the proximal end. The cushion 90 has a lower lip 100 and an upper lip 110.
**FIG. 3** depicts a looped flange 120 on the proximal end of a barrel portion 20 as well as other embodiments.
**FIG. 4** depicts a syringe 10 having a plunger portion 30 having a thumb grip 160 that includes a broken loop positioned on the proximal end.
**FIG.** 5 depicts a plunger portion 30 having a looped thumb grip 180 positioned on the proximal end.
**FIG.** 6 depicts a flow chart describing various possible steps that can be taken during some of the disclosed embodiments.

Throughout the figures, the same reference numerals and characters, unless otherwise stated, are used to denote like features, elements, components or portions of the illustrated embodiments. Moreover, while the subject matter of this application will now be described in detail with reference to the figures, it is done so in connection with the illustrative embodiments. It is intended that changes and modifications can be made to the described embodiments without departing from the true scope and spirit of the subject invention as defined in part by the appended claims.

### DETAILED DESCRIPTION OF THE PREFERRED EMBODIMENTS

In some embodiments, the devices and methods disclosed herein allow for greater ease of use during the manipulation (e.g., injection, aspiration, or aspiration and injection) of a syringe. In some embodiments, the devices and methods allow for greater comfort during use. In some embodiments, the devices and systems disclosed herein allow the user to have an available hand during aspiration and injection to ensure, for example, patient position, tissue tautness, stability, etc. In some embodiments, the devices can be used for stable and accurate one-handed aspiration and injection. In some embodiments, the devices can be used for repeated delivery of a fluid or injectable substance.

It has been realized that the standard injection device, such as a syringe, while adequate for many purposes, has certain shortcomings, especially for certain uses and users. For example, in situations where numerous injections are required an operator can become fatigued or sore from the use of a standard syringe. This can be especially problematic when relatively small volumes are being injected, when multiple injections are made in a relatively short period of time, when the syringe itself is relatively small, or when the precision of the locations of the injections is important (frequently resulting in the user gripping the syringe more tightly). While there are numerous examples of where this can occur (which are not limited to the medical profession or medical treatment), this issue can be especially prominent in the administration of substances such as dermal fillers in cosmetic treatments where many, if not all, of the above issues can be involved at once. In some of the embodiments disclosed herein, these and other considerations have resulted in various ergonomic syringes.

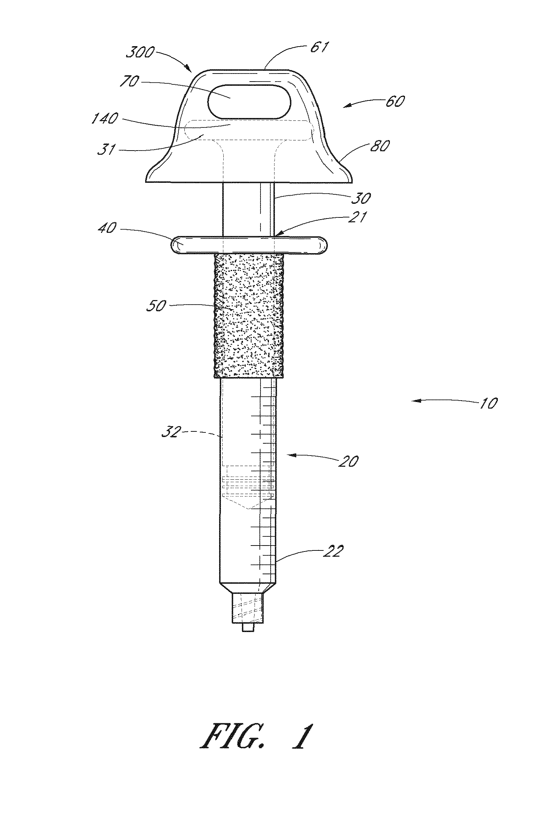

The present description first describes various embodiments of ergonomic syringes with respect to FIGs. 1-5. The description then proceeds to detail alternative embodiments and specific aspects of the ergonomic syringes, subparts, and related devices. Finally, some specific embodiments of using the various devices are disclosed.

FIG. 1 shows some embodiments of a system that can be used to perform a variety of methods or procedures. The syringe 10 has a barrel portion 20 and a plunger portion 30. The plunger portion 30 has a distal end 32 adapted for insertion into a proximal end 21 of the barrel portion 20. In the embodiment illustrated in FIG. 1, the proximal end 21 of the barrel portion 20 has a flange 40 and a cushion 50 adjacent to the barrel portion 20.

In some embodiments, the cushion 50 is configured to provide cushioning when the barrel portion is operably grasped by two adjacent fingers of a same hand. The cushion 50 can be made of a resilient or elastomeric material to provide additional comfort and prevent slippage during operation. In some embodiments, the cushion 50 completely encircles the barrel portion and has a substantially circular cross-sectional area encircling the barrel portion. The illustrated cushion 50 has a textured surface to prevent slipping. Preferably, the cushion 50 is formed of a resilient or elastomeric material.

In some embodiments, the syringe further includes a thumb cushion 60 adjacent to the proximal end of the plunger portion 31. In some embodiments, the size and shape of the syringe 10 is particularly suitable for one-handed aspiration and injection. As will be appreciated by one of skill in the art, while the thumb cushion 60 can be depressed by the thumb, it can also be depressed or acted upon by other parts of the user's hand, such as the palm of the hand.

In some embodiments, the thumb cushion 60 is fitted around the proximal end 31 of the plunger portion 30, or plunger tip, and has a generally oblong cross-sectional area encircling the plunger portion. The thumb cushion 60 has a surface 61 for an operator to press against in order to depress the plunger portion. In some embodiments, the thumb cushion 60 has a lower lip 80 that can act as a flange for aspiration. The lower lip 80 can also be described as a plunger cushion flange 80.

In some embodiments, rather than employing the thumb cushion 60 (having the plunger cushion flange 80 and the surface 61) by pressing on the surface 61, the proximate end of the plunger portion 31 is used as thumb grip 300. The thumb grip 300 can have a hole 70 (which need not be present when only a thumb cushion 60 is desired) generally sized to fit a thumb. The operator can insert a thumb in the hole 70 during operation, and using the thumb, either pull back the plunger portion 30 to aspirate or push down on the plunger portion 30 to inject.

The thumb cushion 60 or thump grip 300 can be made of a soft or resilient material to provide additional comfort and prevent slippage during operation. In the illustrated embodiment, the thumb cushion and thumb grip have a generally oblong cross-sectional area encircling the proximal portion of the plunger portion 30.

In some embodiments, the syringe can further include a flange 40, which can serve as a grip flange for assisting with injections.

As will be appreciated by one of skill in the art, while the thumb cushion and thumb grip are depicted in FIG. 1 as extending past the proximal end 31 of the plunger portion 30, towards the distal end 32 of the plunger portion, it need not extend past the proximal end 31.

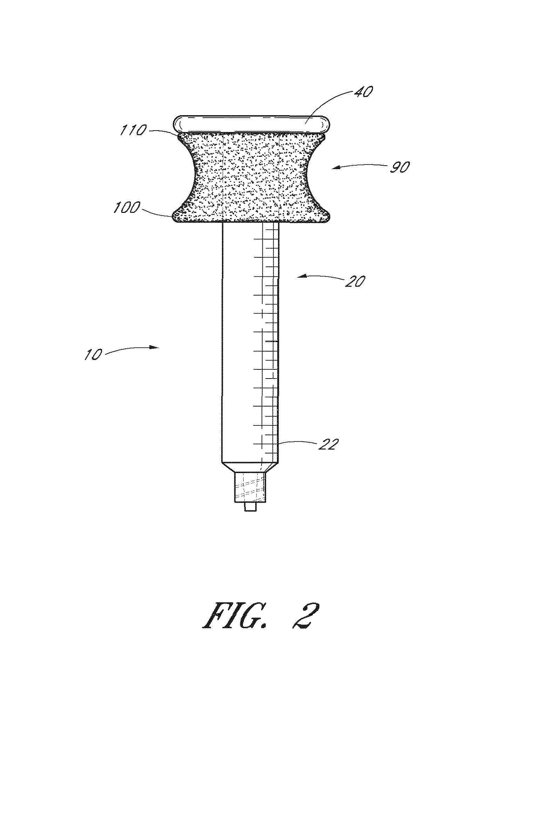

FIG. 2 shows another embodiment of a barrel portion of a syringe. In the illustrated embodiment, a cushion 90 encircles the barrel portion 20 of the syringe 10. The barrel portion 20 also has a circular flange 40.

In the illustrated embodiment, the cushion 90 has a bottom lip 100 and a top lip 110. The bottom lip 100 can also be referred to as a first barrel cushion flange 100. The upper lip 110 can also be referred to as a second barrel cushion flange 110. The barrel cushion flanges 100, 110 can provide an increased surface area and features which, in some embodiments, allow for a more comfortable and secure grip when applying force during manipulation of the syringe 10. These embodiments can also allow one the option of using either one or two hands comfortably. As will be appreciated by one of skill in the art, the cushioning can provide additional comfort for the user's fingers with respect to the barrel portion 20, the flange 40, or both. Additionally, the lower lip 100, can allow for greater ease of use when the user is aspirating the syringe 10 because it can allow for a better grip on the barrel portion 20. In some embodiments, the cushion 90 is made of a soft, resilient, and/or elastomeric material. In this embodiment, the cushion 90 has a generally oblong cross-sectional area encircling the barrel portion 20. The cushion 90 can be configured to provide cushioning when the barrel portion 20 is operably grasped by two adjacent fingers of a same hand. In some embodiments, the cushioning is placed so that at least part of the cushion 90 is positioned between the user's fingers and the barrel portion 20 of the syringe 10. In some embodiments, the generally oblong cross-sectional area of the cushion 90 allows the operator to stabilize, via two adjacent fingers of a same hand, the syringe 10. The cushion 90 can be adjacent to the barrel portion 20, the flange 40, or the barrel portion 20 and the flange 40 during operation. In some embodiments, the flanges 40 in FIGs. 1 and 2 are optional and can be removed. The cushion can provide the gripping surface for the user. Thus, in some embodiments, a syringe that does not have a flange, but does have a cushion, is provided. In some embodiments, the cushion is provided on the opposite side of the flange as depicted in FIGs. 1 and 2. In such embodiments, the barrel can extend past the flange to allow the cushion to be attached to the syringe.

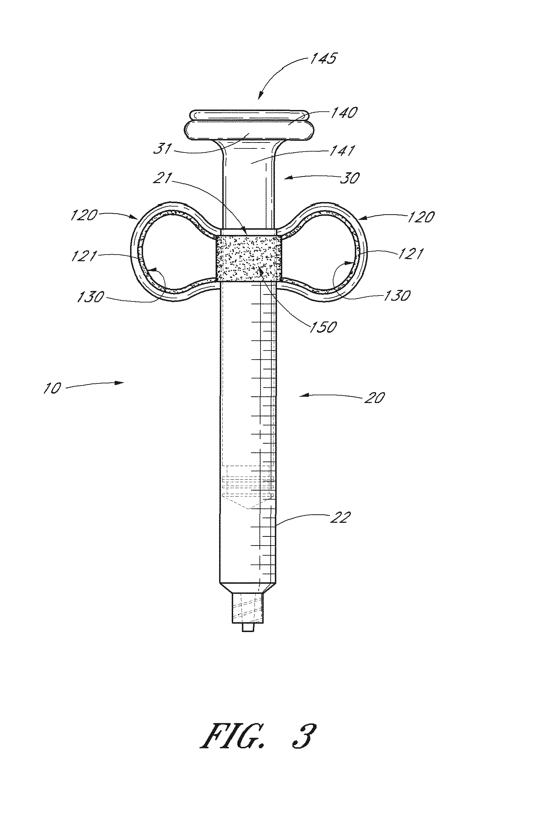

FIG. 3 shows another embodiment of a device that can be used to perform a variety of methods or procedures. In the illustrated embodiment, a closed loop flange 120 is located at the proximal end 21 of the barrel portion 20 of a syringe 10. The closed loop flange 120 has at least one, if not two, finger loops on opposite sides of the barrel portion 20. The closed loop flange 120 can be configured to be grasped by two adjacent fingers of the same hand during use of the syringe 10. As will be appreciated by one of skill in the art, while the closed loop flange 120 is depicted as closed loops, the loops need not be completely closed. In some embodiments, the looped flange 120 is open or broken thereby allowing for greater ease during picking up or discarding the syringe 10 by the user. As will be appreciated by one of skill in the art, while a closed loop flange 120 can provide grip security, it can take more time to grasp and/or release the device. Thus, in some embodiments, a broken or open looped flange is employed instead. This can be especially advantageous when numerous syringes are to be used and/or different syringes interchanged in relatively rapid succession (such as in a single session of injections). As will be appreciated by one of skill in the art, the degree to which the looped flange is "open" can vary. In some embodiments the looped flange is merely cut (and thereby allows a slight increase in flexibility to the structure); however, in some embodiments, more of the loop is removed. In some embodiments, a sufficient amount of the loop is removed to aid in the picking up and release of the syringe. For example, a finger's width of the loop is removed. In some embodiments, two flanges 40 (as depicted in FIG. 1) are used on the barrel portion, with the user's fingers to be placed between the two flanges. In some embodiments, more of the loop is removed, for example, to result in a structure that is similar to that depicted as the cushion flanges in FIG. 2 (110 and 100).

In some embodiments, for example, as depicted in FIG. 3, the plunger portion 30 can have a plunger shaft 141 and a plunger tip 140 located at the proximal end 31. In some embodiments, the plunger tip 140 is formed of a resilient or elastomeric material and can have a larger diameter than the plunger shaft 141, thereby providing a flange on the plunger portion. In some embodiments, the plunger tip 140 is associated with a cushion 145 that is adjacent to the plunger tip 140. It can provide cushioning when the plunger portion 30 is pushed for injection.

In some embodiments, e.g., as depicted in FIG. 3, there is a cushion 121 on the inner surface 130 of each finger loop 120 to provide cushioning when the finger loops are used during pulling and pushing of the plunger portion 30. In some embodiments a cushion 150 is adjacent to the barrel portion 20 of the syringe. In some embodiments, the cushion 150 encircles the barrel portion. In some embodiments, the cushion 150 is in two sections, where each section is primarily located on the barrel portions that are associated with the finger loops 120. In some embodiments, the cushion 150 can provide cushioning when the barrel portion is operably grasped by two adjacent fingers of a same hand.

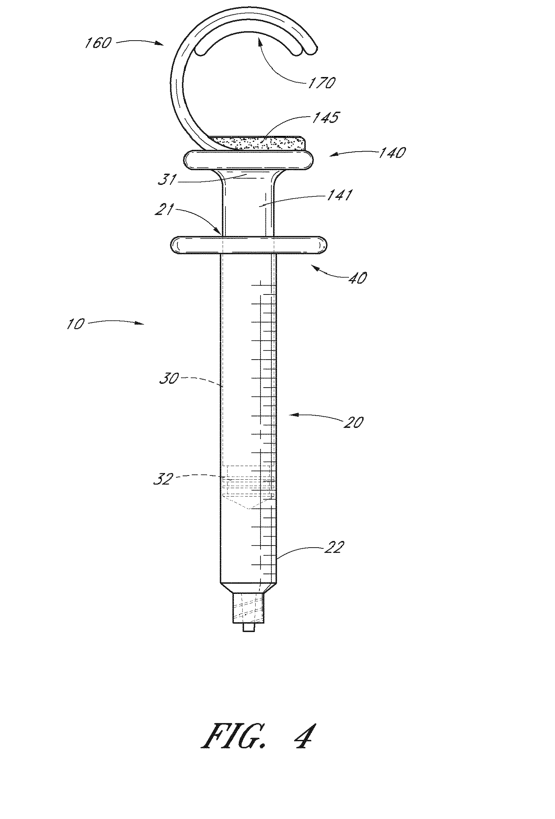

FIG. 4 depicts another embodiment of a system that can be used to perform a variety of methods or procedures. The syringe 10 includes a thumb grip 160 located at the proximal end 31 of the plunger portion 30. In some embodiments, the thumb grip 160 is used to facilitate one-handed aspiration during operation. In the illustrated embodiment, the thumb grip 160 is configured in a partial or broken loop. In some embodiments, a cushion 170 is located on the thumb grip 160 to make use more comfortable. In some embodiments, the cushion 170 is made of a soft, elastomeric or resilient material and is located on the inside surface of the thumb grip 160 to provide a comfortable contact surface when the thumb is used to pull the plunger portion 30 proximally during aspiration.

The plunger portion 30 can include a plunger shaft 141 and a plunger tip 140 located at the proximal end, e.g., as depicted in FIG. 4. In some embodiments, the plunger tip 140 is formed of a resilient or elastomeric material. In some embodiments, an additional cushion 145 can be placed adjacent to the plunger tip 140. In either embodiment, depressing the plunger can be made relatively more comfortable with the cushioning provided by the cushion 145 and/or the cushion 170.

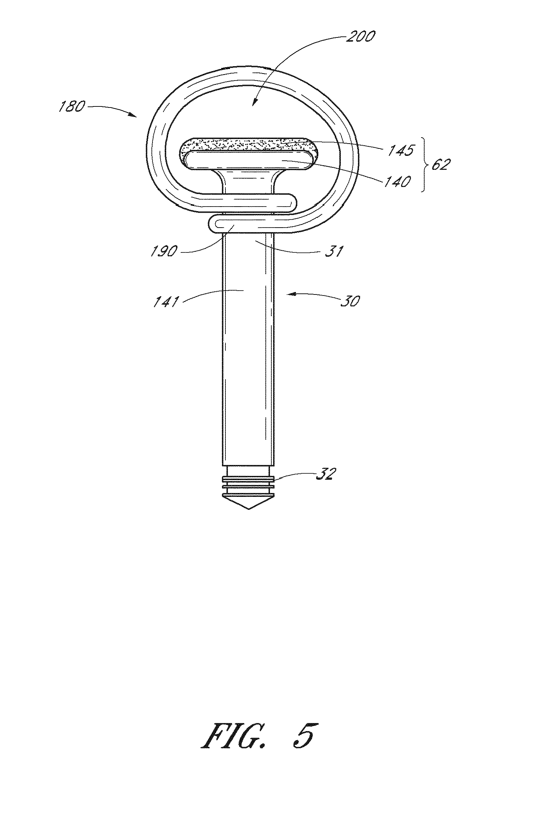

FIG. 5 depicts two alternative embodiments of a plunger portion 30 that can be used to perform a variety of methods or procedures. Those of skill in the art will appreciate that the plunger portion 30 may be used with various embodiments of a barrel portion 20.

In the first embodiment, a band or strap 180 is located at the proximal end 31 of the plunger portion 30 and can encircle the plunger tip 140. This band, strap, or loop can be configured for use as a thumb grip. For example, the band or strap can be sized or sizable to allow a user's thumb to be placed in a gap 200. As with other thumb grips, it can be used to facilitate one-handed aspiration during operation. In some embodiments, the plunger portion 30 can include a plunger shaft 141, and a plunger tip 140 located at the proximal end 31. In some embodiments, the plunger tip 140 is formed of a resilient or elastomeric material. In some embodiments, there is a cushion 145 adjacent to the plunger tip 140. The plunger tip 140 can be pushed by the thumb when injecting.

In some embodiments, the band or strap 180 can be an open or closed loop. In some embodiments, the band or strap 180 can be formed of, for example, a rubber or an elastic band or strip that is wrapped around the plunger tip 140 and secured at the base 190 of the plunger tip 140 by a securing means. The securing means can be, for example, glue, a Velcro^{®} fastener, or a hole in the band through which the plunger shaft 141 is passed. A gap 200 is present between the plunger tip 140 and the band or strap 180. A thumb or finger can be placed in the gap 200. The band or strap 180 can be used to pull the plunger portion 30 proximally to aspirate. The band or strap need not be flexible in all embodiments, although it can be made out of a flexible, elastic, or flexible and elastic material (e.g., rubber, cloth, etc). In such an embodiment, not only can the band allow for easier aspiration, but it can also be relatively comfortable. In some embodiments, an optional cushion 145 is adjacent to not only the plunger tip 145, but can also be adjacent to the inner surface of the band 180. In some embodiments, the loop 180 can be a hard substance, such as a hard plastic. In some embodiments, when the loop formed from the band or strap is smaller than the finger or thumb to be inserted into the loop, the band or strap can be elastic, providing for the extra size of the band or strap when the band or strap is stretched. In some embodiments, the stretched band or strap helps to keep the proximal end of the plunge adjacent to the user's finger or thumb. In some embodiments, this provides for a greater degree of control over the syringe. Further variations on these embodiments are discussed in greater detail below.

In an alternative embodiment of FIG. 5, the band 180 is configured for use as or is used as a thumb cushion 62. In some embodiments, the band 180 can serve as a cushion, can aid in securing a cushion 145 (if such a cushion is present in the embodiment) to the plunger portion 30, or do both. In such an embodiment, while a gap 200 can be present, one of skill in the art will appreciate that it will likely be reduced or eliminated when the plunger is depressed into the barrel portion.

As will be appreciated by one of skill in the art, the band 180 and the cushion 145 need not be placed adjacent to the under side of the plunger tip 140. In some embodiments, one or both are located primarily adjacent to the top (proximal) section of the plunger tip 140.

As will be appreciated by one of skill in the art, any of the above items or figures can be appropriately combined with other items or figures for alternative embodiments. For example, a plunger portion in one figure can be exchanged for any of the other plunger portions in the other figures. Similarly, for example, the barrel portion of FIG. 1 can be replaced with the barrel portion depicted in FIG. 2 or the plunger portion in FIG. 3 can be used in place of the plunger portions in the other figures. The cushion 50 can be used in the embodiments depicted in FIGs. 3 or 4, etc. Similarly, while many of the embodiments described in greater detail below are described in regard to specific applications, one of skill in the art will appreciate that the various embodiments can readily be applied in other situations as well. Thus, the various combinations, unless specifically noted as such, are not to be taken as limiting. The following section generally describes various additional or alternative embodiments and aspects of various embodiments of ergonomic syringes and their use.

### Additional Variations of the Embodiments

Any or all of the following embodiments can be used in isolation or in any or all of the above described embodiments.

In some embodiments, a syringe configured for comfortable, one-handed aspiration and injection is disclosed. In some embodiments, the syringe is configured for one-handed aspiration prior to injection to ascertain that the needle is not in a vessel or other inappropriate anatomical region for the procedure. In some embodiments, the syringe is configured for immediate injection post-aspiration without moving or losing the syringe or needle placement. In some embodiments, the devices and systems disclosed herein provide means for improved accuracy in aspiration and injection compared to two handed aspiration, which can cause significant displacement of the needle.

In some embodiments, the syringes (or parts thereof) disclosed herein are single-use syringes. The syringe can have a barrel portion and a plunger portion having a distal end adapted for insertion into a proximal end of the barrel portion. The barrel portion can include a generally cylindrical, longitudinal passage extending through the barrel portion. In some embodiments, the outer diameter of the barrel portion is less than about 1 cm, for example, 1-0.9, 0.9-0.8, 0.8-0.7, 0.7-0.6, 0.6-0.5, 0.5-0.4, 0.4-0.3, 0.3-0.2 cm or smaller. In some embodiments, the inner diameter of the barrel portion is less than about 1 cm, for example 1-0.9, 0.9-0.8, 0.8-0.7, 0.7-0.6, 0.6-0.5, 0.5-0.4, 0.4-0.3, 0.3-0.2 cm or smaller. In some embodiments, the syringe is small enough to be readily manipulated in one hand and allow for visibility of the barrel portion of the syringe during the manipulation. In some embodiments the inner and outer diameter of the barrel portion can be significantly wider, as it can be desirable to shorten the distal to proximal length of the syringe to accommodate new techniques developed as a result of the greater control and ease of aspiration in using these devices. In some embodiments the inner and outer barrel diameters may be as wide as 2.5 cm, for example, 2.5-2, 2-1.5, 1.5-1, or smaller. In some embodiments, the volume in the syringe is still approximately 2 cc or less even when the syringe is wide.

In some embodiments, the distal end of the barrel portion has an axially extending tip section which can be configured for connection to a Luer lock fitting. The barrel portion can be constructed of a variety of suitable materials, including, for example, glass, metal, and plastic In some embodiments, the material has similar characteristics of expansion and compression, in regard to temperature changes as the syringe. This can help maintain the integrity of the syringe and luer lock fitting during storage or sterilization. Additionally, in some embodiments, the distal end of the barrel has an axially extending tip section for connection directly to a needle, without a leur lock fitting.

The plunger portion 30 can include a plunger shaft 141, and a plunger tip 140 located at the proximal end 31. In some embodiments, the plunger tip 140 is formed of a resilient or elastomeric material. In some embodiments, the distal end of the plunger portion includes a resilient or elastomeric material.

In some embodiments the syringe contains a predefined volume of a substance to be injected, *e.g.*, a flowable substance such as a suspension or liquid. In some embodiments, the substance is one that can be used in cosmetic applications. In some embodiments, this can be a dermal filler, such as RESTYLANE^{®} dermal filler. In some embodiments, the syringe is configured to inject a total volume of less than about 2 cc. In some embodiments, the syringe is configured to inject a total volume of less than about 1 cc. In some embodiments, the syringe contains 2-1.5, 1.5-1, 1-0.5 or less cc of the substance to be injected within the syringe. In some embodiments the substance is botulinum toxin, *e.g.*, botulinum toxin A.

In some embodiments, the syringe is configured to allow for multiple injections of a pre-measured amount of fluid. The pre-measured amount of fluid that can be injected can be in the range of about 0.05 to 0.5 cc. In some embodiments, the pre-measured amount of fluid to be administered is about 0.1 cc to 0.25 cc. In some embodiments, the syringe has stops to indicate when an amount of fluid has been injected. In some embodiments, the stops are visible to an operator when the syringe is gripped between two adjacent fingers.

In some embodiments, the syringe has at least an adaptor such as a cushion, flange, flange grip, thumb grip, loop, or any combination thereof The adaptors can provide for a syringe that can be manipulated with greater ease. In some embodiments, the adaptors allow for a more secure grip when applying force during aspiration or injection. In some embodiments, the adaptors can also make the syringe more comfortable to the operator during use. The adaptors can be for single use or multiple use. In some embodiments, the adaptor is integral to the syringe. In some embodiments, the adaptor is detachable from the syringe. In some embodiments, while the adaptor is not fixed to the syringe, it is located adjacent to the syringe. The adaptor can be attached to the syringe by a variety of means, including, for example without limitation Velcro^{®} fastener, snaps, stretch fit, clamps, glue, tape, rubber cement, adhesives, magnet, mating grooves, interlocking parts, friction, suction, pins, ties, and/or compression fit. In some embodiments, the adaptor is machined to closely fit the syringe. In some embodiments, the adaptor can be sterilized.

Cushions can be positioned in various locations on, against, or associated with the syringe (or other adaptors) to provide a surface for a more secure grip when applying force during aspiration or injection and/or make using the syringe more comfortable. For example, the cushions can be positioned against the barrel portion 20 of the syringe, adjacent to a proximal end of the plunger portion of the syringe, on a thumb grip attached to the plunger portion, adjacent to a flange located on a proximal portion 21 of the barrel portion of the syringe, or any combination of locations thereof. In some embodiments, the syringe has a cushion positioned against the barrel portion 20, wherein the cushion encircles the barrel portion. In some embodiments, the cushion is clipped to the barrel portion 20. In some embodiments, the cushion extends the part way down the barrel from proximal to distal without encircling the barrel.

As will be appreciated by one of skill in the art, in light of the present disclosure, the shape and extent to which a cushion covers a barrel portion or other structure can vary depending upon a number of factors. In some embodiments, the cushion is placed only where needed to distribute forces applied by the fingers. In some embodiments, the cushion completely encircles a barrel portion. In some embodiments, the cushion covers the entire outer surface of the barrel portion (or other structure). In some embodiments, less of the structure is covered. For example, in various embodiments, less than 100%, 100-90, 90-80, 80-70, 70-60, 60-50, 50-40, 40-30, 30-20, 20-10, 10-5, 5-1% or less of the outer surface area of the barrel portion (or other structure) is covered with a cushion. The cushion can be a single cushion or can be separated into numerous parts. For example the cushion could involve two cushions, each on opposite sides of the barrel portion. Any number of cushions could be used, for example, 1, 2, 3, 4, 5, 6, 7, 8, 9, 10, or more cushions can be used.

In some embodiments, the cushioning is removable from the syringe and can be used on different syringes. Thus, in some embodiments, the cushioning can be a removable sleeve that can be used on different syringes. As will be appreciated by one of skill in the art, various embodiments of the cushions described herein can be elastic. In some embodiments, the elastic cushions are attached to the syringe via their elastic properties (for example, the cushion **50**, in FIG. 1 can stay on the syringe because it exerts a constricting force on the barrel of the syringe). Even in such embodiments, in some embodiments, the cushion can still be removable from the syringe. When a cushion is removable from a syringe and covers some of the barrel portion, it can be referred to as a "sleeve" or "sleeved" cushion.

In some embodiments, the cushions can be worn by the user to make using the syringe more comfortable. For example, cushions can be placed on at least a portion of the thumb, fingers, or any combination of thumb and fingers, that come into contact with the syringe or syringe grips. In some embodiments the cushions may be a part of the glove worn by the injector. For example, in some embodiments, the glove is padded in the appropriate locations. For example, the glove can have padding located in positions so that when the syringe is gripped, the padding is placed between the user's fingers or hand and the syringe. In some embodiments, the padding is limited to such locations, so that padding is not present elsewhere in the glove (or only minimally present).

The cushions can be for single use or multiple use. In some embodiments, the cushion is integral to the syringe. In some embodiments, the cushion is detachable from the syringe. The cushion can be attached to the syringe by a variety of means, including, for example without limitation Velcro^{®} fastener, snaps, stretch fit, clamps, glue, tape, rubber cement, hooks, adhesives, magnet, mating grooves, interlocking parts, friction, suction, pins, ties, compression fit. In some embodiments, cushion is machined to closely fit the syringe. The cushion can be sterilized.

The cushion can be made of a variety of materials suitable for providing a relatively soft, relatively comfortable contact surface (for example, in contrast to the glass or hard plastic of the syringe). For example, the cushion can be made of rubber, silicone, cotton, plastic, polymer, various gels, latex, gore-tex (PTFE, EPTFE), thermoplastic elastomers (TPE), neoprene, nitriles, etc. Materials also include the materials used in Dr. Scholl's^{®} brand of gel inserts for shoes or materials of similar rigidity or compressibility. Examples of such materials are disclosed in U.S. Pat. Nos. 7,140,126 and 6,598,321 (the gel disclosures of which are incorporated herein by reference) and include non-foam elastomers such as the class of materials known as viscoelastic polymers or silicone gels. In some embodiments, such gels show high levels of damping when tested by dynamic mechanical analysis performed in the range of -50° C to 100° C. In some embodiments, the mechanical properties of the gel can be more viscous than elastic and the gel provides a high energy absorption. Additional gels that can be used are thermoplastic elastomers (elastomeric materials), such as materials made from polymeric families, including but not limited to the Kraton family of styrene-olefin-rubber block copolymers, thermoplastic polyurethanes, thermoplastic poly olefins, polyamides, polyureas, polyesters and other polymer materials that reversibly soften as a function of temperature. Kraton block copolymer of styrene/ethylene-co-butylene/styrene or styrene/butadiene/styrene with mineral oil incorporated into the matrix as a plasticizer can also be used in a cushion.

Preferably, the cushions are made of a material providing a resilient or yielding surface. In some embodiments, the cushions provide comfort and/or prevent slippage when using the syringe. In some embodiments, the cushions are made of rubber and have a textured surface. The textured surface can provide a surface for a more secure grip when applying force during aspiration or injection. The textured surface can also provide for greater comfort during manipulation of the syringe. In some embodiments the cushion is or comprises an elastic component.

The cushions can be of any thickness suitable for providing a comfortable surface on the syringe or adaptor. In some embodiments, the cushion is between about 1 mm to about 1.5 cm thick. In some embodiments, the cushion has a thickness of about 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15 mm or any amount on either side of or defined between the aforementioned thickness values. A cushion can have varying thicknesses. For example, a portion of the cushion can be about 0.5 cm thick, and another portion of the cushion can be about 1 cm thick.

As will be appreciated by one of skill in the art, in light of the present disclosure, the cushions can be yielding, compressible, cushioning, and/or deformable to a sufficient extent to provide for some increase in comfort, grip, or both (as desired or required). As will be appreciated by one of skill in the art, the amount of the above characteristic, such as compressibility, will vary depending upon the particular location of the cushion and the various factors involved in the use that the syringe is to be implemented in. For example, applications in which precision of volume injected is less important (or mitigated by other factors) will allow for a greater amount of deformability to be present. Similarly, applications where precision of the location of the injections are of relatively less importance will also allow materials with greater deformability to be used. When such factors are of more importance, one would generally favor cushions with less deformability. Thus, depending upon user preference and the actual use of the device, the cushions can have a compressibility of any amount, for example from less than 1% to 99% or more, including 1-2, 2-3, 3-5, 5-8, 8-10, 10-15, 15-20, 20-30, 30-40, 40-50, 50-60, 60-70, 70-80, 80-90, 90-95, 95-99 or more during the use of the syringe. This amount can be determined by measuring the initial height or thickness of a cushion while it is not being used (e.g., while no force is being applied) and comparing it to the height or thickness of the cushion while it is being used. Thus, a cushion that changes thickness by 50% when it is being used has a compressibility of 50%. Rubber and similar substances can be measured for "softness" according to Shore (Durometer) tests. In some embodiments, the cushion is between 20 and 95 on Shore A scale or up to about 50 on a Shore D scale. In some embodiments the cushion is up to 60 on the Shore A scale. In some embodiments, the cushion is below 40, 30, or 20 on the Shore A scale. However, other values above and below these can be used in some embodiments. As will be appreciated by one of skill in the art, substantial comfort can be obtained with minimal compressibility. For example, the cushion can compress less than 5%, 5-2, 2-1, 1-0.5, 0.5-0.1, or less. Additionally, not all of the cushions have to be compressible or deformable. In some embodiments, the padding or cushions simply add comfort for the user. In some embodiments, the cushion adds comfort for the user without requiring that the cushion be deformable or measurably compressible.

In some embodiments, the cushions are at least partially transparent. In some embodiments, the cushions are sufficiently transparent to allow a user to view the amount of substance being administered when the plunger is depressed. However, depending upon the placement of the cushion, the amount of transparency can vary and can be less than 100%, for example, 100-90, 90-80, 80-70, 70-60, 60-50, 50-40, 40-30, 30-20, 20-10, 10-1, or less. In some embodiments, the cushions are sufficiently transparent so as to allow a user to read the volume indicators on the syringe and/or identify the tip of the plunger with respect to these volume indicators.

In some embodiments the cushion has a substantially circular cross-sectional area encircling the barrel portion or plunger portion of the syringe. In other embodiments, the cushion has a generally oblong cross-sectional area encircling the barrel portion or plunger portion of the syringe. The cushion can have an upper lip, a lower lip, or an upper lip and a lower lip. The lips can provide increased surface area and features when desired to enable a more secure grip when applying force during injection and or aspiration. In some embodiments, at least a portion of a cushion positioned against a barrel portion extends at least about 0.5 cm beyond the circumference of the barrel portion. In some embodiments, at least a portion of a cushion adjacent to a proximal end of the plunger portion of the syringe extends at least about 0.5 cm beyond the circumference of the plunger portion. In some embodiments, the cushion is deformable, has shape memory, or compressible.

In some embodiments the syringe includes a thumb grip 300, 160, 180, *e.g.*, a loop or ring sized (or sizable) for a thumb. The thumb grip can be used to facilitate one-handed aspiration during operation. In some embodiments, the thumb grip is located at a proximal end 31 of the plunger portion 30 of a syringe 10. The thumb grip can be in a closed loop or in an open configuration. In some embodiments, the thumb grip is a broken loop. In some embodiments, the thumb grip includes a cushion having a hole generally sized to fit a thumb. In some embodiments, the thumb grip includes an elastic band configured to fit around the plunger tip. In some embodiments, the elastic band encircles the plunger tip. In some embodiments the band is hard plastic and attaches through interlocking compression design.

In some embodiments, the thumb grip can be rigid or flexible. The thumb grip can be made of a variety of materials, including without limitation, rubber, cloth, plastic, elastic, glass, metal, neoprene, gore-tex, silicone, TPE, nylon, cotton, foam rubber, the materials discussed above in regard to the cushion, or any combination thereof. In some embodiments, the thumb grip functions as a cushion. In some embodiments, the thumb grip functions as an aspirating loop, so as to allow one to pull the plunger out of the barrel. As will be appreciated by one of skill in the art, while this is called a "thumb" grip, other fingers can be used in this grip.

In some embodiments, a cushion is located on the thumb grip to make use more comfortable. In some embodiments, the cushion is configured to provide a comfortable surface while pushing down on the plunger portion. In some embodiments, the cushion is configured to provide a comfortable surface when the thumb grip is used to pull back on the plunger portion. In some embodiments, the thumb grip is used to pull the plunger portion proximally to aspirate. In other embodiments, the thumb grip is used to push the plunger portion distally to inject. In other embodiments, the thumb grip is used to both push the plunger portion distally to inject and alternately to pull the plunger proximally to aspirate.

The thumb grip can be for single use or multiple use. In some embodiments, the thumb grip is integral to the syringe. In some embodiments, the thumb grip is detachable from the syringe. The thumb grip can be attached to the syringe by a variety of means, including, for example without limitation Velcro^{®} fastener, snaps, stretch fit, clamps, holes, glue, tape, rubber cement, hooks, adhesives, magnet, mating grooves, interlocking parts, friction, suction, pins, ties, compression fit. In some embodiments, the thumb grip is machined to closely fit the syringe. In some embodiments, the thumb grip can be sterilized or sterilizable.

In some embodiments, the thumb grip includes, comprises, consists, or consists essentially of, a flexible, elastic, or flexible and elastic strap that is or can be configured to fit around the distal end of the plunger 32 and then slid up to the proximal end of the plunger 31 to its position of use. The strap can be made of a variety of materials, including without limitation, rubber, cloth, plastic, elastic, glass, metal, neoprene, gore-tex, silicone, TPE, nylon, cotton, foam rubber, or any combination thereof. In some embodiments, the strap encircles the plunger tip.

In some embodiments, the strap is sized so that, when attached to the proximal end of the plunger, it forms a loop that encompasses the plunger tip 140 and that is the size of a user's finger or thumb. In some embodiments, the strap forms a loop that is smaller than the size of the user's finger or thumb that will manipulate the plunger. However, as the strap can be elastic, the strap can stretch to encompass the user's finger (which can include thumb) and stably associate that plunger with the user's finger.

In some embodiments, the strap can have at least one hole through which the distal end of the plunger portion of a syringe can be passed, thereby attaching the strap on the plunger portion of the syringe. For example, the strap can be attached to the plunger portion by insertion of a distal end of the syringe through a hole at each end of the strap, thus forming a loop around a proximal end of the plunger portion (for example, FIG. 5). The loop is preferably generally sized to fit a thumb or finger (if the material is elastic, then the stretched loop can be the part that is appropriately sized). The operator can insert a thumb or finger in the loop during operation, and either pull back the plunger portion to aspirate, via the strap, or push down on the plunger portion to inject.

The strap can be of any length suitable for allowing a user's thumb or finger to be inserted between the proximal end of the plunger portion and the strap (in either the stretched or unstretched arrangements). In some embodiments, the strap is between about 2 cm to about 6 cm long. In some embodiments, the strap has a length of 1.0, 1.5, 2.0., 2.5, 3.0, 3.5, 4.0, 4.5, 5.0, 5.5, 6.0, 6.5, 7, 7.5, 8, 8.5, 9, 9.5, 10, 10.5 cm or a length of less than or defined between any of the aforementioned length values. In some embodiments where the strap is formed of an elastic material, the length of the strap can increase during stretching. Depending on the elasticity of the material, the length of the strap can increase, for example from less than 1% to 100% or it can increase from more than 1 to 500% or more, including, for example, 1-2, 2-3, 3-5, 5-8, 8-10, 10-15, 15-20, 20-30, 30-40, 40-50, 50-60, 60-70, 70-80, 80-90, 90-95, 95-99, 99-100, 100-150, 150-200, 200-300, 300-400, 400-500 or more when the finger inserted into the loop. In some embodiments, the strap has a length of between 1-10, 2-8, 3-8, 4-8, 4-7, or 5-6 cm when stretched in use on the proximal end of the plunger. The "in use" values listed above denote the length of the strap when the finger is inserted into the loop, not when the finger is aspirating the syringe (which, in some embodiments, can further increase the length of the strap).

The strap can be of any width suitable for providing support for the thumb or finger during syringe use. In some embodiments, the strap is between about 0.5 mm to about 4 cm wide. In some embodiments, the strap has a width of about 0.5 1, 1.5, 2, 2.5, 3 or any amount on either side of or defined between the aforementioned length values. In some embodiments, where the strap is formed of an elastic material, the width of the strap can increase or decrease during stretching. In some embodiments, the strap can have varying width. For example, a portion of the strap can be about 1 cm wide, and another portion of the cushion can be about 1.5 cm wide.

In some embodiments, the strap can be of any thickness suitable for providing a comfortable surface for the thumb or finger during aspiration, for keeping the proximal end of the plunger close to the user's finger or thumb, or for both purposes. In some embodiments, the strap is between about 0.1 mm to about 1 cm thick. In some embodiments, the cushion has a thickness of about 0.1, 0.5, 1, 1.5, 2, 2.5, 3, 3.5, 4, 4.5, 5, 5.5, 6, 6.5, 7, 7.5, 8, 8.5, 9, 9.5, 10 mm or any amount on either side of or defined between any one of the aforementioned thickness values. A strap can have varying thicknesses. For example, a portion of the strap can be about 1 mm thick and another portion of the cushion can be about 3 cm thick. In some embodiments where the strap is formed of an elastic material, the thickness of the strap can decrease during stretching.

In some embodiments, a method of making a syringe involving a thumb grip is provided. In some embodiments, the method involves a strap (any one of the herein described straps) having a hole at both ends of the strap. The distal end of a plunger portion of the syringe can be inserted through each hole and the strap slid up to the proximal end of the plunger portion, where it can be restrained from falling off of the plunger. This restraint can be achieved by the plunger portion itself. For example, the proximal end (or the plunger tip 140) can be larger than the holes in the strap, thereby preventing the strap from sliding off of the plunger. The plunger can then be inserted in to a proximal portion of a barrel portion of a syringe. The insertion of the plunger portion through both holes in the strap can result in the formation of a loop around the proximal end of the plunger portion, thereby forming a loop for insertion of a user's thumb or finger.

As will be appreciated by one of skill in the art, when holes (which includes slits or types of openings) are used to attach the thumb grip to the plunger, the diameter of at least one of the holes should be smaller than the diameter of widest section of the proximal end of the plunger portion or the plunger tip, thereby allowing the widest section to prevent the thumb grip from coming off the syringe.

In some embodiments, a cushion is located on or within the thumb grip to make use more comfortable. In some embodiments, the cushion is configured to provide a comfortable surface while pushing down on the plunger portion. In some embodiments, the cushion is configured to provide a comfortable surface when the thumb grip is used to pull back on the plunger portion. In some embodiments, the thumb grip is used to pull the plunger portion proximally to aspirate. In other embodiments, the thumb grip is used to push the plunger portion distally to inject. In other embodiments, the thumb grip is used to both push the plunger portion distally to inject and alternately to pull the plunger proximally to aspirate.

In some embodiments, a method of using a thumb grip is provided. In some embodiments the method comprises providing a syringe comprising a barrel portion and a plunger portion comprising a distal end adapted for insertion into a proximal end of the barrel portion and providing a strap that is looped around a proximal end of the plunger portion. In some embodiments, the strap comprises, consists, or consists essentially of a first hole and a second hole. In some embodiments at least a part of the plunger portion is passed through the first hole and second hole. The strap is sized (either when stretched or not) to allow a finger or thumb to be inserted between the proximal end of the plunger portion and the strap. The strap can comprise a flexible, elastic, or flexible and elastic material. One can then insert a finger or thumb between the proximal end of the plunger and the strap and aspirate the syringe by exerting a force against the strap via the finger or thumb. In some embodiments, when the finger or thumb is inserted between the proximal end of the plunger and the strap, the strap is stretched. In some embodiments, the stretched strap exerts a constricting force on the finger or thumb.

In some embodiments the syringe includes a flange 40 on the proximal portion of the barrel portion of the syringe 10. The flange can be configured to be grasped by two adjacent fingers of the same hand. In some embodiments, the flange is circular. In some embodiments, the flange has two portions, each portion on opposite sides of the barrel portion. In some embodiments the flange 40 is rigid. In some embodiments, the flange can be grasped by two adjacent fingers of the same hand. In some embodiments, the flange is rotatable around the barrel portion. The flange can be integral to the barrel portion. In some embodiments, the flange is detachable from the barrel portion. The flange can completely encircle the barrel portion in some embodiments. In other embodiments, the flange partially encircles the barrel portion. In some embodiments, the flange is configured to be clipped onto the barrel portion.

The flange can extend about 1 mm to about 5 cm or more out from the barrel portion. In some embodiments, the flange extends about 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15 mm, or any amount on either side of or defined between the aforementioned values out from the barrel portion. The flange can be of varying thickness, and is preferably of a comfortable thickness when grasped by two adjacent finger of the same hand. In some embodiments, the flange has a thickness of about 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15 mm, or any amount on either side of or defined between the aforementioned thickness values.

In some embodiments, the flange includes finger loops to secure the fingers on the flange during pulling and pushing of the plunger portion. A finger loop can be rigid or flexible. The finger loop can be closed loops or in an open configuration. In some embodiments, the finger loop is a broken ring. The finger loop can be made of a variety of materials, including without limitation, rubber, cloth, plastic, elastic, metal, glass, neoprene, goretex, silicone, TPE, or any combination thereof.

In some embodiments, a cushion is located on the flange or finger loops to make use more comfortable. In some embodiments, a cushion is located adjacent to the flange and configured to provide cushioning when a force is exerted against the flange.

The flange can be rigid or flexible. In some embodiments, the flange is rigid. In some embodiments, the flange is rotatable. The flange can be made of a variety of materials, including without limitation, rubber, cloth, plastic, elastic, metal, glass, silicone, neoprene, goretex, TPE, or any combination thereof. In some embodiments, the flange is made of rubber and has a textured surface.

The flange can be configured for single use or multiple use. In some embodiments, the flange is integral to the syringe. In some embodiments, the flange is detachable from the syringe. The flange can be attached to the syringe by a variety of means, including, for example without limitation Velcro^{®} fastener, snaps, stretch fit, clamps, glue, tape, rubber cement, hooks, adhesives, magnet, mating grooves, interlocking parts, friction, suction, pins, ties, compression fit. In some embodiments, the flange is machined to closely fit the syringe. The thumb grip can be sterilized. A syringe can include more than one flange, located on either or both of the barrel portion or the plunger portion or cushions associated therewith.

As will be appreciated by one of skill in the art, when a cushion is associated or adjacent to a particular syringe structure, there can be embodiments in which there are additional intervening structures between the cushion and the syringe. For example, additional layers of padding, plastic, tape, attachment means, coatings, epoxy or glue, or other structures can be placed between the cushion and the syringe without preventing the cushion from being "associated with" or "adjacent to" the syringe or barrel portion. In embodiments in which there is necessarily nothing between the syringe or barrel portion and the cushion, the cushion can be described as being immediately adjacent, directly associated, or similar phrase.

In some embodiments, various aspects can be included together as a kit. In some embodiments, the kit includes at least two syringes. Each syringe can include the following: a barrel portion, a cushion positioned against the barrel portion (the cushion is configured to provide cushioning when the barrel portion is operably grasped by two adjacent fingers of a same hand), a plunger portion having a distal end adapted for insertion into a proximal end of the barrel portion, and a specified amount of a dermal filler contained within the barrel portion. In some embodiments, the barrel portion contains about 1 cc or less of dermal filler. In some embodiments, 3, 4, 5, 6, 7, 8, 9, 10, or more syringes are packaged together.

In some embodiments the kit includes at least one syringe. The syringe has a barrel portion and a plunger portion. The plunger portion has a distal end adapted for insertion into a proximal end of the barrel portion. The kit also has a cushion configured to be positioned against the barrel portion. The cushion can provide cushioning when the barrel portion is operably grasped by two adjacent fingers of one hand. In some embodiments there is a specified amount of a dermal filler contained within the barrel portion. In some embodiments, the cushion is integral to the barrel portion of the syringe. In some embodiments, the cushion is detachable from the syringe. In other embodiments, the cushion is configured to be worn by the syringe operator. In some embodiments, the cushion is configured to be attached to a finger of the syringe operator, and a second cushion is configured to be attached to an adjacent finger of the same hand. In some embodiments, the cushion has an adhesive layer on the side to be positioned against the syringe, allowing one to apply the cushion to the syringe. In some embodiments the cushion is a part of a glove. In some embodiments the plunger is packaged separate from the barrel to be inserted during use. In some embodiments the plunger, barrel, are packaged pre-assembled.

In some embodiments, the kit or syringe is further associated with a needle. In some embodiments the needle is already attached to the syringe. In some embodiments, the needle is configured for the administration of a dermal filler. In some embodiments, the needle is configured for the administration of a dermal filler to the facial region of a subject. In some embodiments, the needle is smaller than a 23 gauge needle, for example 23-25, 25-27, 27, 27-28, 28-31, 28-29, 29-30, 30, 30-31 or smaller. In some embodiments, the needle is a 30G1/2 sized needle. In some embodiments, the needle is between 0.5 to 1.5 inches in length.

In some embodiments the kit includes at least one syringe and at least one strap (such as any strap described herein). In some embodiments the strap has a first hole at a first end and a second hole at a second end of the strap. In some embodiments, the kit further includes a cushion positioned against the barrel portion.

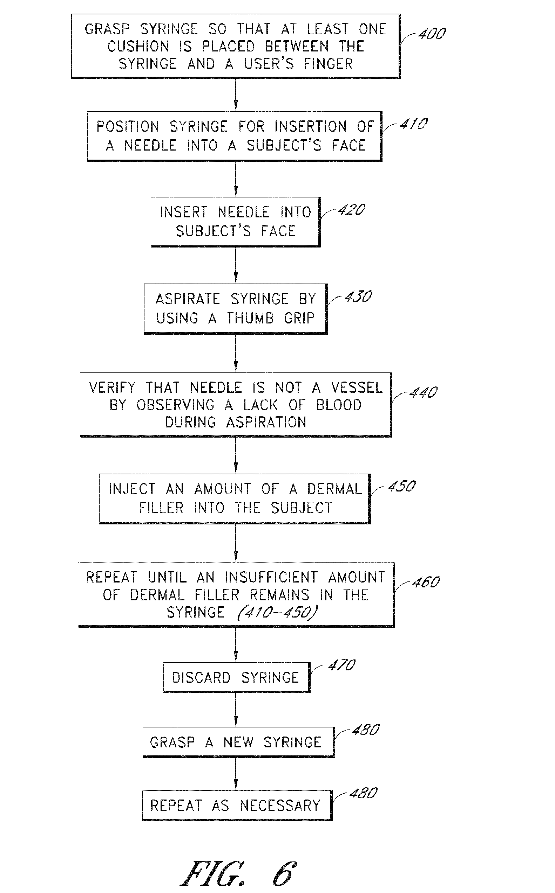

In some embodiments, a method of comfortably administering a liquid can be achieved by using one or some of the above embodiments. FIG. 6 depicts one embodiment of a method of using some embodiments of an ergonomic syringe. Not all of the steps need be included and not all of the below described steps have been described in the figure.

In some embodiments, a user can grip a syringe 10, (*e.g.*, as disclosed in FIGs. 1-3) so that at least a portion of the cushion 50, 90, 150 is positioned between the user's fingers and the syringe 10 (as shown in step 400). In some embodiments, the user grips the syringe 10 by holding, gripping, or pressing onto or against the cushion. In some embodiments, the user holds the syringe via the inner sides of two adjacent fingers. In some embodiments, the user holds the syringe using primarily the index finger and the middle finger. In some embodiments, the user holds the syringe in a single hand.

The user can then manipulate the syringe for placement of the syringe (step 410) and insertion of the needle into the subject (step 420). In some embodiments, the user uses a single hand to do one or both of these operations. In some embodiments, the user uses two fingers to position and insert the needle of the syringe into the subject. In some embodiments the syringe is gripped by the index and middle fingers during the insertion of the needle into the subject. In some embodiments the needle is inserted into a location that will benefit from the injection of a dermal filler. In some embodiments, the injections are made to the face of a subject.

In some embodiments, the user can then optionally aspirate the syringe to verify that the needle has not been inserted into a portion of the body, into which injection of the liquid is not desired (*e.g.*, for injection of a dermal filler, a vein or artery (step 430)). In some embodiments, this is done by withdrawing the plunger portion slightly and observing if blood is taken into the syringe or needle (step 440). In some embodiments, this is achieved by using the thumb of the hand holding the syringe and withdrawing the plunger via the thumb grip. If too much blood is observed during aspiration (to indicate that the needle was inserted into a blood vessel), the needle can be withdrawn from the subject and inserted elsewhere.

In some embodiments, aspiration is simplified by including a flange, thumb hook, or thumb grip on the plunger. In some embodiments, the thumb grip is a loop (opened or closed). In some embodiments, the thumb grip has a cushion associated with it. Thus, in some embodiments, while one operates the thumb grip, a cushion is located between at least a portion of the user's hand (which includes fingers) and a remaining part of the syringe. Thus, in an embodiment where one uses a plunger portion 30 as shown in FIG. 4, the padding 170 is placed between the location where the thumb is to be positioned and the rest of the thumb grip 160. In some embodiments, the cushion is the entire thumb grip itself; thus, aspiration can involve simply manipulating the cushion, for example, by moving the cushion (or strap) away from the syringe body. Such a method would include embodiments involving the use of the plunger portions depicted in FIG. 5, or the optional hole shown in FIG. 1. As will be appreciated by one of skill in the art, in some embodiments, a finger can be used to operate the thumb grip instead of a thumb. In some embodiments while one operates the thumb grip, the thumb grip may actually be against the heel of the operator's hand.

In some embodiments, the user can then depress the plunger portion to inject the flowable substance into the subject (step 450). In some embodiments, the user at least grips the syringe by the inside portion of two adjacent fingers on one hand while depressing the plunger portion. In some embodiments, at least a portion of at least one cushion will be located between at least one of the user's fingers and the body of the syringe or the plunger of the syringe. By compressing the syringe, the user will transfer stress or force to the cushion, which can alter the distribution of the forces felt by the user so that a more comfortable and/or secure feeling grip will result. In some embodiments, the plunger is depressed by the user's thumb. In some embodiments, the plunger is depressed by another part of the user's hand, such as the palm of the hand.

In some embodiments, the user will inject a substance related to cosmetics. For example, the user can inject a dermal filler. In some embodiments, the substance is a dermal filler and the placement of the injection is determined by where an amount of dermal filler should be injected. In some embodiments, the substance is RESTYLANE® hyaluronic acid derivative dermal filler. In some embodiments, the substance is PERLANE® hyaluronic acid derivative dermal filler. In some embodiments, less than 2 cc of the substance is injected, for example 2-1.5, 1.5-1, 1-0.5, 0.5-0.4, 0.4-0.3, 0.3-0.2, 0.2-0.1, 0.1-0.05, 0.05-0.01 cc, or less. As will be appreciated by one of skill in the art, the method need not be limited to cosmetic or medical issues as any application of a syringe could benefit from certain embodiments of the described invention. For example, in some embodiments, the substance is glue and is injected into cracks in wood that are to be repaired.

In some embodiments, the user repeats the above steps (e.g., steps 410-450) multiple times with a single syringe (e.g., step 460). In some embodiments, the user repeats the above steps 3 to 20 times with a single syringe. In some embodiments a single syringe can be used to make 3-8 injections (and optional aspirations) in a face of a subject. In some embodiments, the user uses multiple syringes in a single treatment session. For example, the user can use between 2 and 50 syringes in any one session. In some embodiments, the user disposes of each syringe when the syringe is empty (step 470). In some embodiments, the user then grasps a new syringe that is prefilled with the substance (step 401). In some embodiments the substance is a dermal filler. In some embodiments, approximately 0.1 cc of the substance is injected with each proper insertion of the needle and approximately 5-10 pre-filled syringes are used on a single subject in one sitting. As will be appreciated by one of skill in the art, the above steps can be repeated as necessary in order to achieve the desired goal (step 480).

In some embodiments, the use of the thumb grip allows for single-handed aspiration in order to determine if the needle is in a vessel. In some embodiments, the use of the thumb grip allows for immediate injection, postaspiration, without moving and/or losing the syringe and/or needle placement. In some embodiments, the use of the disclosed device can result in an improvement in the aspiration/injection approximation, as two-handed aspiration can result in significant displacement of the needles. Thus, in some embodiments, use of the device as described herein allows for a method having some or all of the above described advantages.

In some embodiments, the user can apply the cushions to the hands or fingers, rather than using cushions that are fixed to the syringe. For example, the cushions can be placed on the user's fingers, or gloves, or be a part of the gloves, especially the index and middle fingers and especially on the inside of these two fingers. Similarly a cushion can be placed on the user's thumb. The cushion can include substances such as band-aids, tape, foam tape, adhesives, gels, etc. Preferably, the cushion does not interfere with the use of gloves.

The various devices and systems described above provide a number of ways to carry out the invention. It is to be understood that not necessarily all objectives or advantages described can be achieved in accordance with any particular embodiment described herein. Also, although the invention has been disclosed in the context of certain embodiments and examples, it will be understood by those skilled in the art that the invention extends beyond the specifically disclosed embodiments to other alternative embodiments and/or uses and obvious modifications and equivalents thereof. Accordingly, the invention is not intended to be limited by the specific disclosures of preferred embodiments herein. Unless denoted otherwise in the above specification by an explicitly recited exclusion, embodiments described as "including," "having," "comprising," etc., are also contemplated as consisting and consisting essentially of the recited elements. This only applies to the use of these terms in the specification and does not apply to the use of the terms in the claims.

## Claims

1. A syringe, comprising:
a barrel portion (20);
a plunger portion (30) comprising a distal end adapted for insertion into a proximal end of said barrel portion (20); and
a gripping device (160) positioned on a proximal end (31) of said plunger portion, the gripping device comprising an open loop portion that is sized and configured to operatively receive a user's thumb and to facilitate one-handed aspiration during operation of the syringe.

2. The syringe of claim 1, further comprising a predefined volume of a dermal filler contained within said barrel portion (20).

3. The syringe of claim 1, wherein said syringe is configured to inject a total volume of less than about 2.0 ml.

4. The syringe of claim 1, wherein said syringe is configured to inject a total volume of less than about 1 ml.

5. The syringe of claim 1, wherein an outer diameter of the barrel portion (20) is less than about 1 cm.

6. The syringe of claim 1, wherein said syringe further comprises a cushion (170) positioned on said gripping device (160) and configured to provide cushioning during one-handed aspiration.

7. The syringe of claim 1, wherein said gripping device (160) is integral to the syringe.

8. The syringe of claim 1, wherein said gripping device is detachable from the syringe.

9. The syringe of claim 1, wherein said syringe is configured to inject multiple, pre-measured amounts of fluid, wherein said pre-measured amount is about 0.1 ml.

10. The syringe of claim 1, wherein said syringe further comprises stops to indicate when a pre-measured amount of fluid has been injected, and wherein said stops are visible to an operator when the syringe is gripped between two adjacent fingers.

11. The syringe of claim 1, further comprising a flange (40) located on a proximal portion of the barrel portion (20).

12. The syringe of claim 1, wherein the syringe is sterilizable.

13. The syringe of claim 1, wherein the open loop portion comprises a first end and a second end, and an opening between the first end and the second end to enable a user to place their thumb through the opening.
